Europäisches Patentamt

European Patent Office

Office européen des brevets

(11) Publication number: **0 145 339**
**B1**

(12) **EUROPEAN PATENT SPECIFICATION**

(45) Date of publication of patent specification: **25.05.88**

(51) Int. Cl.⁴: **A 61 F 2/32**

(21) Application number: **84307970.8**

(22) Date of filing: **16.11.84**

(54) Acetabular prosthesis.

(30) Priority: **25.11.83 US 555165**

(43) Date of publication of application:
**19.06.85 Bulletin 85/25**

(45) Publication of the grant of the patent:
**25.05.88 Bulletin 88/21**

(84) Designated Contracting States:
**CH DE FR GB LI**

(56) References cited:
**EP-A-0 051 599**
**DE-A-3 130 732**
**FR-A-2 266 491**
**US-A-3 863 273**
**US-A-3 992 725**
**US-A-4 180 873**
**US-A-4 266 303**
**US-A-4 318 191**
**US-A-4 362 681**
**BIOCOMPATIBLE POLYMERS, March 18, 1983,
pages 975-979, (US), H. LANDFIELD:
"Sterilization of medical devices based on
polymer selection and stabilization
techniques"**

(73) Proprietor: **VITEK INC.**
**3143 Yellowstone Road**
**Houston Texas 77054 (US)**

(72) Inventor: **Homsy, Charles Albert**
**11526 Raintree Circle**
**Houston Texas 77024 (US)**

(74) Representative: **Lambert, Hugh Richmond et al
D. YOUNG & CO. 10 Staple Inn
London, WC1V 7RD (GB)**

Courier Press, Leamington Spa, England.

## Description

The present invention relates to an improved acetabular prosthesis for use in a total hip joint prosthesis which uses materials that conform and adapt to the shape of the natural acetabulum of the patient.

A total hip prosthesis commonly includes two components: a femoral-head prosthesis and an acetabular prosthesis.

A known femoral-head prosthesis includes a ball-type head which has the shape of a hemisphere. A stem extends from the head and is adapted for insertion into a femoral medullary canal. The stem is preferably coated with a stabilizing material, either an acrylic cement or a low modulus tissue ingrowth material, such as is disclosed in US—A—3,992,725.

One known type of acetabular prosthesis includes a hemispherical cup which is fixed within the natural acetabulum with an acrylic cement and through anchoring holes in the pelvis. Screws or the like extend from the convex side of the cup and engage the holes in the pelvis.

But, when the cement non-uniformly transfers load stresses, there can result a loss of bone leading to a gradual degradation of the useful life of the implanted prosthesis.

An acetabular prosthesis is also known which has a metallic hemispherical cup which is not mechanically secured to the pelvis. The cup has an internal concave surface which is lined with a biocompatible and wear resistant polymer such as ultra high molecular weight polyethylene. Articulation in this implant takes place on one hand between the head of the femoral-head prosthesis and the lining of the cup, and on the other hand between the polished convex surface of the cup and the natural acetabulum.

While such an unfixed acetabular prosthesis does not require holes in the pelvis, it may develop serious problems due to the fact that the exterior of the cup is hemispherical and the natural acetabulum in which the cup articulates is not a true portion of a sphere because it may have natural or diseade-caused irregularities, which can cause loads transmitted through the hip joint to become localised and abnormal, thereby damaging the natural acetabulum.

This problem is described in US—A—4,318,191, wherein a unitary hip joint prosthesis is described having a compliant metal head which is intended to conform and adjust to the shape of the natural acetabulum and thereby to avoide abnormal stress loadings. This metal head leaves much to be desired as to its ability to adjust and protect the acetabulum against abnormal dynamic laods which are normally transmitted by the hip joint.

In US—A—4,362,681 an acetabular prosthesis is disclosed in which the cup member is coated on its outer surface with a rigid, foamed or sintered porous coating of a selected bioengineering thermoplastics which is said to have a strength like bone, and which promotes tissue ingrowth. The coating has a thickness of from 0.5 to 10 mm, an average pore diameter of from 90 to 600 microns, pore connections having average diameters greater than 50 microns, a modulus of elasticity of from 250,000 to 3,000,000 p.s.i. (1,724 to 20,685 MPa), a porosity of greater than 25%, and a total creep stain of less than 1% at 1,000 p.i.s. (6.9 MPa) at ambient temperature. Such rigid bioengineering thermoplastics, which support the applied load without assistance from ingrowth bone, and without pore distortion (which limits tissue ingrowth), is specifically contrasted with, and advocated over high density polyethylene and polytetrafluoroethylene composites in which significant changes in pore structure occur under compressive stresses as low as 80 p.s.i. (552 kPa) for polyethylene, and 300 p.s.i. (2069 kPa) for polytetrafluoroethylene graphite composites. The porous bioengineering thermoplastics used in accordance with the teachings of US—A—4,362,681 are therefore essentially rigid, load-bearing materials which maintain their porosity under load, and which by virtue of their rigid, non-compressible load-bearing characteristics, do not solve the problem of providing for uniform load bearing over the whole implant. Moreover the user of such rigid material may tend to damage the softer cartilage of the acetabulum.

The present invention therefore seeks to provide an improved acetabular prosthesis which avoids non-uniform and abnormal load transmission to the natural acetabulum, which does not require pelvic fixation, and which maintains a uniform engagement with the surface of the natural acetabulum.

This is achieved in accordance with the present invention, and in specific contrast to the teachings of US—A—4,362,681, by providing on the cup a surface layer of a compressible porous biocompatible material of sufficient thickness and sufficient deformability and limited elastic memory to allow it to mould itself into the particular anatomic geometry of the acetabulum. In general, the coating will have, in its uncompressed state, a porosity in the range from 40% to 90%, preferably about 60%, and a thickness in the range from 1 to 10 mm. Preferably the coating will also have a plurality of grooves on its exterior surface to allow, in use, bulk movement of synovial fluid therethrough. In specific contrast with the teachings of US—A—4,362,681, such a compressible, deformable layer will, when the joint is loaded, undergo a considerable degree of compression and pore distribution, as well as moulding itself to the exact surface contour of the acetebulum, and thereby provide for uniform load distribution.

This invention is further described in connection with the accompanying drawings wherein:

Figure 1 is a partial sectional view of a hip joint prosthesis featuring the improved acetabular prosthesis of this invention, and illustrating the relative positions of the femur and pelvis.

Figure 2 is a plan view of the external surface of the acetabular prosthesis shown in Figure 1.

Figure 3 is a partial sectional view of a modified form of the acetabular prosthesis shown in Figure 1.

Figure 4 is a partial sectional view of another modified form of the acetabular prosthesis shown in Figure 3.

With particular reference now to the drawings, the hip joint prosthesis, generally designated as 10, includes two components: a femoral-head prosthesis 12, and an acetabular prosthesis 14.

Prosthesis 12 is preferably of the type described in GB—A—2,118,441. It includes a polished hemisphere ball 16, a neck 18, and a stem 20, which has a coating 22 bonded to its exterior surface. Coating 22 is preferably a tissue ingrowth material, such as is disclosed in said patent US—A—3,992,725. Stem 20 is inserted into medullary canal 24 of femur 26. Coating 22 serves to stabilize or fix prosthesis 12 in femur 26.

Prosthesis 14 includes a cup 28 which is made of a sufficiently strong material which can be an implantable metal, polysulfone, polycarbonate, or a polyacetal polymer. Prosthesis 14 is inserted into the acetabulum 27a of pelvis 27. The internal surface of cup 28 is provided with a lining 30, and the external surface of cup 28 is covered by a coating 32. Lining 30 is preferably made of a suitable biocompatible wear-resistant material, such as ultra-high molecular weight polyethylene. Lining 30 is suitably secured within cup 28 by a press fit. Lining 30 has an interior surface 34 which has at least in part a hemispherical shape and in which ball 16 articulates.

Coating 32, which has a thickness in the range of 1 to 10 mm, is a deformable material having little or low elastic memory. Coating 32 may be a coating of porous polytetrafluoroethylene exhibiting a porosity in the range from 40% to 90%; 60% porosity is preferred. Coatings having a thickness in the upper end of said range are used for a natural acetabulum having variations which require such thickness for the self-moulding of the coating therein, i.e. for the coating to deform and adapt itself to the natural shape of acetabulum 27a. It is preferred that the porous polytetrafluoroethylene coating 32 be a material prepared in accordance with the disclosure in EP—A—0,051,955. Coating 32 is bonded to the exterior surface of cup 28 by a suitable bonding agent. Fluorinated ethylene propylene is a preferred bonding agent for the preferred coating 32. Another appropriate material for coating 32 is the material disclosed in US—A—3,992,725. Such material can be also bonded to cup 28 by the same bonding agent as the preferred material.

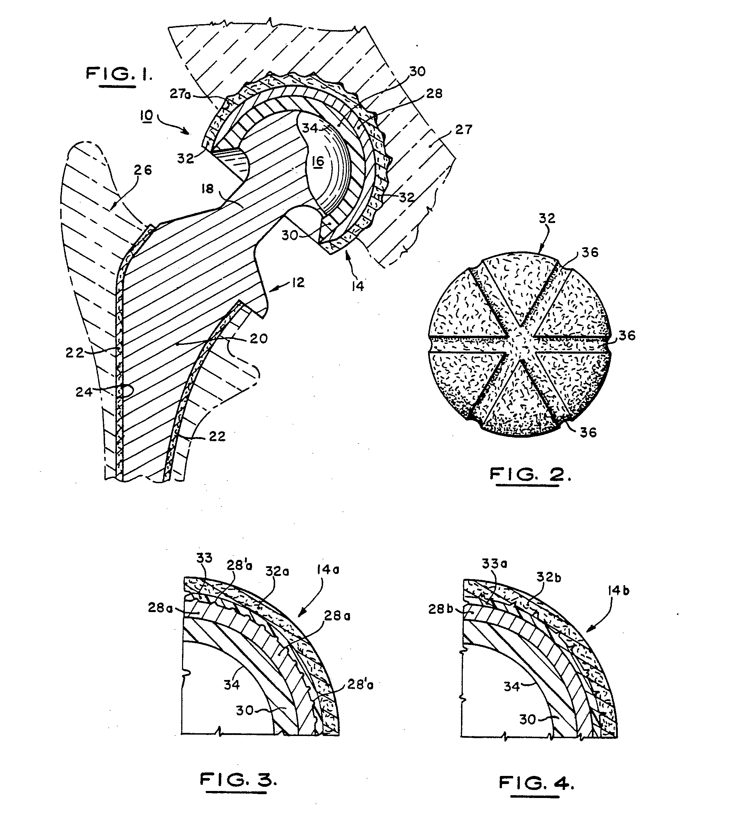

As shown in Figure 2, coating 32 preferably includes a plurality of rounded grooves 36 on its exterior surface, to allow synovial fluid to flow therethrough. The low or limited elastic memory of coating 32, even when compressed, allows it to be compliant with the apposed natural cartilage so that the coating's deformation continuously accommodates the topography of the cartilage. In the deformed state of coating 32, further loading is attenuated by the elasticity of coating 32 and of the cartilage, thereby simulating continuous impact absorption as in a natural joint. Further, this uniform loading allows the joint to function almost as a natural joint. Also the normal perfusion of nutrients into the cartilage assists in maintaining the natural health of the joint.

In Figure 3 is shown a modified acetabular prosthesis 14a which includes a cup 28a with an interior lining 30 and an exterior coating 32a, as shown and described with reference to Figure 1. However, coating 32a is not bonded directly to the exterior surface of cup 28a but to an intermediate layer 33 of medical grade elastomer. Attachment of layer 33 to cup 28a is by mechanical interlocking of the elastomer to a roughness 28'a on the exterior surface of cup 28a, and attachment of layer 33 to coating 32a is by mechanical interlocking to the surface porosity of coating 32a.

Another modified acetabulum prosthesis 14b, shown in Figure 4, is similar to the embodiment of Figure 3, except that a portion of coating 32b, adjacent to the exterior surface of cup 28b, is now impregnated with a medical grade elastomer 33a. Attachment of the impregnated coating 32b to cup 28 is by mechanical interlocking of the elastomer with the surface of cup 28b. In order to achieve this structure, the portion of coating 32b which is impregnated with the medical grade elastomer is made to have a porosity of 70% to 90%. The remainder of coating 32b exhibits a porosity in the range from 40% to 90%; 60% porosity is preferred.

In the modified forms of FIGURES 3 and 4, it is preferred that the medical grade elastomer be a suitable medical grade silicone. These modified forms provide additional resiliency to the prosthetic joint which will aid in the absorption of shock energy during ambulation of the patient.

The low or limited elastic memory of coating 32, even when compressed, allows it to be compliant with the apposed natural cartilage so that the deformation of the cartilage is the means of perfusing nutrients into the cartilage.

The modified acetabular prostheses 14a and 14b accommodate local deviations on the order of 1.0 mm from the non-hemispherical shape of the natural acetabulum 27a. The larger end of the thickness range for coating 32 allows it to self-mold to an overall distorted shape of the natural acetabulum. Although coating 32 has a low elastic memory in order to deform and mold itself to the acetabular cavity, it should preferably also possess a low degree of elasticity which would allow it to absorb energy during the cyclic impositions of loads thereon.

**Claims**

1. An acetabular prosthesis comprising a substantially hemispherical cup-shaped insert (14) insertable as a friction fit in the acetabulum and providing a substantially hemispherical socket opening adapted to receive the ball (16) of a femoral head prosthesis (12), said cup-shaped member (28, 28a, 28b) defining on its inside said hemispherical socket and being coated on its outer surface with a layer (32, 32a, 32b) of porous biocompatible material having a thickness of from 1 to 10 mm, and which, upon insertion of the insert into the acetabulum makes contact with the surface contour of the acetabulum, characterised in that said layer (32, 32a, 32b) is a compressible layer of porous bicompatible material having a limited elastic memory, and which, upon insertion of the insert, moulds itself to conform with irregularities in the surface contour of the acetabulum, thereby to make uniform surface contact therewith, said layer, in the uncompressed state, having a porosity in the range 40% to 90%.

2. A prosthesis according to claim 1, characterised in that said cup-shaped member (28, 28a, 28b) is made from metal, polysulfone polymer, polycarbonate polymer or polyacetal polymer.

3. A prosthesis according to claim 1 or 2, characterised in that the inner surface of said cup-shaped member is lined with a lining (30) of wear resistant material.

4. A prosthesis according to claim 3, characterised in that said lining (30) is of ultra-high molecular weight polyethylene.

5. A prosthesis according to any one of claims 1—4, characterised in that said layer (32, 32a, 32b) of porous, biocompatible material, in the uncompressed state, has a porosity of approximately 60%.

6. A prosthesis according to any one of claims 1—5, characterised in that said porous biocompatible material of limited elastic memory is porous polytetrafluoroethylene.

7. A prosthesis according to any one of claims 1—6, characterised in that said layer (32, 32a, 32b) of porous, biocompatible material has a plurality of grooves (36) on its exterior surface to allow, in use, bulk movement of synovial fluid therethrough.

8. A prosthesis according to any one of claims 1—7, characterised in that said layer (32) of porous, biocompatible material is adhesively bonded to said cup-shaped member (28).

9. A prosthesis according to any one of claims 1—7, characterised in that a layer (33, 33a) of medical grade elastomer is located between the external surface of said cup-shaped member (28a, 28b) and said layer (32a, 32b) of porous, biocompatible material.

10. A prosthesis according to claim 9, characterised in that said layer (33) of elastomer is mechanically interlocked to said cup-shaped member (28a) and to the layer (32a) of porous, biocompatible material.

11. A prosthesis according to claim 9, characterised in that said layer (33a) of elastomer is keyed to said layer (32b) of porous biocompatible material by impregnation into the pores thereof.

12. A prosthesis according to claim 9, 10 or 11, characterised in that said elastomer is a medical grade silicone.

**Patentansprüche**

1. Hüftgelenkpfannenprothese mit einem im wesentlichen halbkugelförmigen, becherförmigen Einsatz (14), der als ein Reibungssitz in der Hüftgelenkpfanne einsetzbar ist und eine im wesentlichen halbkugelförmige Pfannenöffnung liefert, die so ausgebildet ist, daß sie die Kugel (16) einer Oberschenkelkopfprothese (12) aufnimmt, wobei das tassenförmige Teil (28, 28a, 28b) auf seiner Innenseite die halbkugelförmige Pfanne definiert und auf seiner äußeren Oberfläche mit einer Schicht (32, 32a, 32b) aus porösem biologisch verträglichem Material mit einer Dicke von 1 bis 10 mm beschichtet ist und beim Einfügen des Einsatzes in die Hüftgelenkpfanne eine Berührung mit dem Oberflächenumriß der Hüftgelenkpfanne herstellt, dadurch gekennzeichnet, daß die Schicht (32, 32a, 32b) eine zusammenpreßbare Schicht eines porösen biologisch verträglichen Materials mit begrenztem elastischem Rückstellvermögen ist, die beim Einfügen des Einsatzes sich selbst so formt, daß sie sich an die Unregelmäßigkeiten in dem Oberflächenumriß der Hüftgelenkpfanne anpaßt und so einen gleichmäßen Oberflächenkontakt mit ihr herstellt, wobei die Schicht im nichtzusammengepreßten Zustand eine Porosität im Bereich von 40% bis 90% hat.

2. Prothese nach Anspruch 1, dadurch gekennzeichnet, daß das becherförmige Teil (28, 28a, 28b) aus Metall, Polysulfonpolymer, Polycarbonatpolymer oder Polyacetatpolymer besteht.

3. Prothese nach Anspruch 1 oder 2, dadurch gekennzeichnet, daß die innere Oberfläche des becherförmigen Teils mit einer Auskleidung (30) aus verschließbeständigen Material ausgekleidet ist.

4. Prothese nach Anspruch 3, dadurch gekennzeichnet, daß die Auskleidung (3) aus ultrahochmolekularem Polyethylen besteht.

5. Prothese nach einem der Ansprüche 1 bis 4, dadurch gekennzeichnet, daß die Schicht (32, 32a, 32b) aus porösem, biologisch verträglichem Material im nichtzusammengepreßten Zustand eine Porosität von etwa 60% hat.

6. Prothese nach einem der Ansprüche 1 bis 5, dadurch gekennzeichnet, daß das poröse, biologisch verträgliche Material mit begrenztem elastischem Rückstellvermögen poröses Polytetrafluorethylen ist.

7. Prothese nach einem der Ansprüche 1 bis 6, dadurch gekennzeichnet, daß die Schicht (32, 32a, 32b) aus porösem, biogolisch verträglichem Material mehrere Nuten (36) auf ihrer äußeren Oberfläche hat, um bei der Verwendung eine Massenbewegung von Synovialflüssigkeit durch sie hindurch zu gestatten.

8. Prothese nach einem der Ansprüche 1 bis 7, dadurch gekennzeichnet, daß die Schicht (32) aus porösem, biologisch verträglichem Material haftend an das becherförmige Teil (28) gebunden ist.

9. Prothese nach einem der Ansprüche 1 bis 7, dadurch gekennzeichnet, daß eine Schicht (33, 33a) aus einem Elastomer von medizinischer Qua lität zwischen der äußeren Oberfläche des becherförmigen Teils (28a, 28b) und der Schicht (32a, 32b) aus porösem, biologisch verträglichem Material angeordnet ist.

10. Prothese nach Anspruch 9, dadurch gekennzeichnet, daß die Schicht (33) aus Elastomer mechanisch mit dem becherförmigen Teil (28a) und der Schicht (32a) aus porösem, biologisch verträglichem Material verblockt ist.

11. Prothese nach Anspruch 9, dadurch gekennzeichnet, daß die Schicht (33a) aus Elastomer mit der Schicht (32b) aus porösem, biologisch verträglichem Material durch Imprägnierung von dessen Poren verkeilt ist.

12. Prothese nach Anspruch 9, 10 oder 11, dadurch gekennzeichnet, daß das Elastomer ein Silicon von medizinischer Qualität ist.

## Revendications

1. Prothèse cotyloïdienne comprenant un implant en forme de coupelle (14), sensiblement hémisphérique à insérer par ajustage à frottement dans le cotyle et offrant une ouverture en douille sensiblement hémisphérique propre à recevoir la rotule (16) d'une prothèse de tête de fémur (12), ledit élément en forme de coupelle (28, 28a, 28b) définissant sur son intérieur ladite douille hémisphérique et étant revêtu sur sa surface extérieure d'une couche (32, 32a, 32b) de matériau biocompatible poreux ayant une épaisseur de 1 à 10 mm, et qui, lors de l'insertion de l'implant dans le cotyle entre en contact avec le contour superficiel du cotyle, caractérisée en ce que ladite couche (32, 32a, 32b) est une couche compressible de matériau biocompatible poreux ayant une mémoire élastique limitée, et qui, lors de l'insertion de l'implant, se moule pour se conformer aux irrégularités du contour superficiel du cotyle, pour entrer par là en contact superficiel avce celui-ci, la dite couche ayant, à l'état non comprimé, une porosité comprise entre 40% et 90%.

2. Prothèse selon la revendication 1, caractérisée en ce que ledit élément en forme de coupelle (28, 28a, 28b) est en métal, polymère de polysulfone, polymère de polycarbonate ou polymère de polyacétal.

3. Prothèse selon la revendication 1 ou 2, caractérisée en ce que la surface intérieure dudit élément en forme de coupelle porte un garnissage (30) en matériau résistant à l'usure.

4. Prothèse selon la revendication 3, caractérisée en ce que ledit garnissage (30) est en polyéthylène à poids moléculaire extrêmement élevé.

5. Prothèse selon l'une quelconque des revendications 1 à 4, caractérisée en ce que ladite couche (32, 32a, 32b) en matériau biocompatible poreux a, à l'état non comprimé, une porosité d'environ 60%.

6. Prothèse selon l'une quelconque des revendications 1 à 5, caractérisée en ce que ledit matériau biocompatible poreux à mémoire élastique limitée est du polytétrafluoréthylène poreux.

7. Prothèse selon l'une quelconque des revendications 1 à 6, caractérisée en ce que ladite couche (32, 32a, 32b) en matériau biocompatible poreux présente plusieurs gorges (36) sur sa surface extérieure pour permettre, en service, un déplacement en bloc du fluide synovial à travers celles-ci.

8. Prothèse selon l'une quelconque des revendications 1 à 7, caractérisée en ce que ladite couche (32) de matériau biocompatible poreux est liée par adhérence audit élément en forme de coupelle (28).

9. Prothèse selon l'une quelconque des revendications 1 à 7, caractérisée en ce qu'une couche (33, 33a) d'élastomère de qualité médicale est située entre la surface extérieure dudit élément en coupelle (28a, 28b) et ladite couche (32a, 32b) de matériau biocompatible poreux.

10. Prothèse selon la revendication 9, caractérisée en ce que ladite couche (33) d'élastomère est mécaniquement verrouillée sur ledit élément en coupelle (28a) et sur la couche (32a) de matériau biocompatible poreux.

11. Prothèse selon la revendication 9, caractérisée en ce que ladite couche (33a) d'élastomère est clavetée sur la dite couche (32b) de matériau biocompatible poreux par imprégnation des pores de celui-ci.

12. Prothèse selon la revendication 9, 10 ou 11, caractérisée en ce que ledit élastomère est un silicone de qualité médicale.

FIG. I.

FIG. 2.

FIG. 3.

FIG. 4.